# FASCICULE DE BREVET EUROPEEN

(11) **EP 0 576 323 B1**
(45) Date de publication et mention de la délivrance du brevet: **10.09.1997**
(21) Numéro de dépôt: 93401492.9
(22) Date de dépôt: 10.06.1993
(51) Int. Cl.: C07C 2/60

(54) **Procédé amélioré d'alkylation d'hydrocarbures aliphatiques**
Verfahren zur Alkylierung von aliphatischen Kohlenwasserstoffen
Process for the alkylation of aliphatic hydrocarbons

(30) Priorité: 24.06.1992 FR 9207860
(43) Date de publication de la demande: 29.12.1993
(73) Titulaire: INSTITUT FRANCAIS DU PETROLE, 92502 Rueil-Malmaison (FR)
(72) Inventeur: Chauvin, Yves, F-78230 Le Pecq (FR); Commereuc, Dominique, F-92190 Meudon (FR); Olivier, Hélène, F-92500 Rueil Malmaison (FR); Hirschauer, André, F-78360 Montesson (FR)

(56) Documents cités:
- FR-A- 2 626 572
- US-A- 4 357 482

## Description

L'objet de la présente invention est un procédé de production d'hydrocarbures paraffiniques par réaction d'addition d'oléfines sur des isoalcanes en présence de catalyseurs de Friedel-Crafts modifiés.

On connait un grand nombre de catalyseurs acides, liquides ou solides, susceptibles de réaliser l'alkylation d'isoparaffines telles que l'isobutane ou l'isopentane, par des oléfines telles que le propylène, les butènes-1 ou -2, I'isobutène. Les catalyseurs les plus souvent utilisés dans la pratique industrielle sont l'acide sulfurique concentré, l'acide fluorhydrique, seul ou en mélange avec des acides de Lewis tels que le trifluorure de bore. On a également préconisé l'emploi de catalyseurs solides: les aluminosilicates tels que les zéolithes, ou les oxydes métalliques tels que la zircone traités par des acides forts, comme l'acide sulfurique. Le chlorure d'aluminium, quant à lui, a été utilisé pour l'alkylation de l'isobutane par l'éthylène.

Bien qu'assez répandus en raison de l'utilisation de plus en plus importante des isoparaffines dans les carburants pour moteurs à allumage commandé, ces procédés souffrent d'inconvénients préjudiciables à leur généralisation: l'acide fluorhydrique à cause de sa toxicité et de sa volatilité; l'acide sulfurique à cause d'une consommation importante nécessitant un retraitement onéreux. Les catalyseurs solides quant à eux, se sont jusqu'à présent montrés peu sélectifs et peu actifs.

Il a été proposé, dans la demande de brevet français n° 2.626.572 d'utiliser pour catalyser l'alkylation paraffinique, les complexes liquides que font les halogénures d'aluminium avec certains halogénures d'ammonium quaternaires, complexes qui ne sont pas miscibles avec les paraffines. Ces complexes, dits "sels fondus", sont avantageusement utilisés pour l'alkylation des isoparaffines telles que l'isobutane avec des oléfines telles que l'éthylène, le propylène, les butènes-1 et-2.

Il a maintenant été trouvé que les complexes que font les halogénures d'aluminium avec les chlorhydrates ou les bromhydrates d'amines, complexes qu'on qualifie également de "sels fondus", présentent une activité et une sélectivité encore plus importante que les complexes à base de sels d'ammonium quaternaires pour les réactions d'alkylation. Un exemple de tels complexes a été décrit par C. A. Angell et J. W. Shuppert dans J. Phys. Chem; 84 , 538, 1980.

Les halogénures d'aluminium plus particulièrement utilisables selon l'invention sont le trichlorure ou le tribromure d'aluminium, qui peuvent être mis en oeuvre seuls ou en mélange, dans des proportions variables.

Les chlorhydrates ou les bromhydrates d'amines plus particulièrement utilisables selon l'invention, comportent une mole d'acide halohydrique par mole d'amine, mais peuvent également comporter deux moles d'acide par mole d'amine. On peut aussi utiliser des mélanges d'halohydrates à une mole et à deux moles d'acide halohydrique. Les halohydrates dérivent d'amines ou de diamines acycliques ou d'amines faisant partie d'un cycle qui peut comporter un ou plusieurs atomes d'azote, et répondant aux formules générales suivantes: 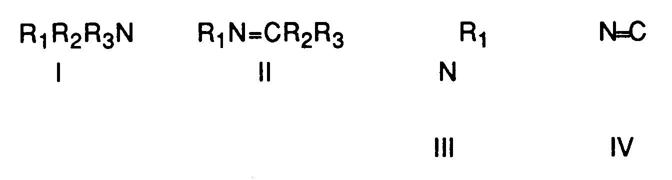 dans lesquelles R₁, R₂, R_{3,} identiques ou différents, représentent des restes hydrocarbyles, par exemple alkyl, cycloalkyl, aryl, aralkyl ou alkaryl. Les cycles III et IV sont contitués de 5 à 10 atomes, de préférence de 5 à 6 atomes qui peuvent comprendre, outre un ou des atomes d'azote, des atomes de carbone liés par des liaisons simples ou doubles. Les cycles III et IV peuvent être condensés avec d'autres cycles et porter des substituants hydrocarbonés tels que définis ci-dessus, des fonctions amines, des atomes de fluor, de chlore, de brome.

Parmi les groupements R₁ _{,} R₂ et R₃ on mentionnera les radicaux méthyl, éthyl, propyl, isopropyl, butyl, sec-butyl, amyl, méthylène, éthylidène, phényl, benzyl. Les cycles tels que IV sont représentés par les familles des pyridines, des imidazoles, des triazines, des pyrazoles, des pyrimidines, des triazoles

Comme exemples d'amines dont les chlorhydrates ou les bromhydrates font partie des complexes selon l'invention on peut citer plus particulièrement la pyridine, les picolines- 2, -3 ou -4, les lutidines, l'éthyl-2 pyridine, l'isopropyl-3 pyridine, la chloro-4 pyridine, la chloro-2 pyridine, la N,N-diméthylamino-4 pyridine, le N- méthylimidazole, le N-butyl imidazole, la pipéridine, la N-méthylimidazoline.

Les proportions relatives d'halogénures d'aluminium et d'halohydrates d'amines déterminent à la fois l'acidité (ou la basicité) du milieu et son point de fusion. Quand la fraction molaire d'halogénure d'aluminium est inférieure à 0,5 le mélange est très peu acide, voire basique et l'activité catalytique est faible, voire nulle. Si elle est supérieure à cette valeur le milieu est d'autant plus acide que cette fraction molaire est plus grande. On dispose donc d'une gamme d'acidité très étendue qu'on peut faire varier en fonction de la réactivité de l'oléfine qui est mise en oeuvre, ce qui a pour avantage de minimiser les réactions parasites d'oligomérisation, tout en maximisant la vitesse de réaction. Ainsi dans le cas de l'éthylène, la moins réactive des oléfines, on choisira de préférence une fraction molaire comprise, par exemple, entre 0,6 et 0,7; pour le propylène et les n-butènes une fraction molaire comprise entre 0,49 et 0,6 peut se révéler préférable. En tout état de cause, on choisira une composition liquide à la température de la réaction.

Ainsi, l'invention concerne un procédé d'alkylation d'au moins une isoparaffine par au moins une oléfine en présence d'un catalyseur renfermant au moins un halogénure d'aluminium, caractérisé en ce que le catalyseur est constitué par un mélange liquide à la température de réaction d'au moins un halogénure d'aluminium avec au moins un halohydrate d'une amine, la fraction molaire d'halogénure d'ammonium étant supérieure à 0,5.

L'alkylation est conduite en présence de deux phases liquides : le "sel fondu" d'une part, et la phase hydrocarbonnée contenant les réactifs et les produits. Une agitation vigoureuse est nécessaire pour assurer un bon contact entre le catalyseur et les réactifs.

La réaction peut être conduite en système fermé (les réactifs et le catalyseur étant introduits en totalité en début de réaction), en système semi-ouvert (les, ou un des réactifs étant introduits au fur et à mesure de l'avancement de la réaction), ou en système ouvert (les réactifs, et éventuellement de la phase catalytique ou un des composants de la phase catalytique, sont introduits en continu; les produits, et éventuellement une partie de la phase catalytique, étant soutirés en continu). En système fermé ou semi-ouvert, on arrête l'agitation quand on le juge utile, on laisse se séparer les deux phases et on soutire la phase hydrocarbonée; une nouvelle charge pourra être introduite. Quand on opère en système ouvert, l'étage de réaction est suivi d'un étage de décantation qui permet de séparer les deux phases, la phase catalytique étant en totalité ou en partie retournée au réacteur. Les deux étages peuvent être inclus dans le même récipient.

Quand le catalyseur a perdu une partie notable de son activité on peut le réactiver en ajoutant un des constituants du "sel", par exemple de l'halogénure d'aluminium et/ou de l'acide halohydrique et ou un halogénure d'alkyle.

L'isoparaffine, isobutane ou isopentane par exemple, et l'oléfine telle que l'éthylène, le propylène et/ou les butènes, sont mis en oeuvre dans des proportions telles que le rapport molaire alcane/oléfine soit compris entre 2 et 100, de préférence compris entre 3 et 30.

On peut traiter les isoparaffines seules ou en mélange, ainsi que les oléfines seules ou en mélange.

Le rapport du volume de la phase catalytique au volume de la phase hydrocarbonnée peut varier dans de très larges limites allant de 100 : 1 à 1 : 100 de préférence de 20 : 1 à 1 : 20. On déterminera pour chaque cas le rapport conduisant, pour un volume global donné, aux résultats les meilleurs.

La température de réaction sera comprise entre -30° C et +70° C, de préférence entre -10° C et +30° C, une température élevée favorisant la vitesse de réaction mais également les réactions secondaires, en particulier la formation d'oligomères et de produits halogénés organiques ou organominéraux.

On impose une pression telle qu'au moins la majeure partie de la paraffine réactive se trouve dans la phase liquide, mais la pression n'est pas limitée et on peut imposer une pression telle qu'il n'y ait pas de phase gazeuse.

Les exemples suivants illustrent l'invention sans en limiter la portée.

### EXEMPLE N° 1

*Préparation du chlorhydrate de pyridine.* Dans un ballon à fond rond contenant 60 mL d'ether sec et 0,1 mole de pyridine on fait buller lentement de l'acide chlorhydrique gazeux. Il se forme quantitativement un précipité de chlorhydrate de pyridine (point de fusion : 145° C)

*Préparation d'un sel acide.* Dans une suspension de 24,8 g de chlorure d'aluminium dans de l'heptane on ajoute 0,1 mole de chlorhydrate de pyridine. On observe la formation rapide d'une phase ionique liquide non miscible avec l'heptane.

*Alkylation de l'isobutane par l'éthylène.* Dans un tube en verre à pression, maintenu à 20°C, on introduit 6 g du "sel" préparé précédemment et 33 g d'isobutane contenant 2 g de n-butane (étalon interne). On pressurise avec de l'éthylène jusqu'à 0,9 MPa, pression que l'on maintient constante par ajout d'éthylène. Après 3 hr d'agitation 43% de l'isobutane initialement présent a été converti. Le produit de la réaction a été soutiré; il avait la composition suivante:
méthylbutane: 5,2 %
diméthyl-2,3 butane: 74,4 %
autres isohexanes: 5,6 %
triméthyl-2,2,4 pentane: 4,6 %
autres isooctanes: 8 %
alcanes supérieurs: 2 %

On a recommencé 15 fois la réaction avec des résultats comparables. A la quinzième fois l'activité du catalyseur avait décrue. On lui a appliqué une pression d'acide chlorhydrique anhydre de 0,2 MPa. Après dégazage de l'acide en excès on a pu répéter la réaction d'alkylation avec les mêmes résultats que précédemment.

### EXEMPLE N° 2

*Préparation du chlorhydrate de picoline-4.* Dans un ballon à fond rond contenant 60 mL d'éther sec et 0,1 mole de picoline-4 on fait buller lentement un courant d'acide chlorhydrique gazeux. Il se forme un précipité cristallin (point de fusion: 170° C).

*Préparation d'un sel acide.* Dans une suspension de 7,83 g de chlorure d'aluminium dans de l'heptane on ajoute 0,031 mole de chlorhydrate de picoline. On observe la formation rapide d'une phase ionique non miscible.

*Alkylation de l'isobutane par l'éthylène.* Dans un tube en verre à pression, maintenu à 20° C, on introduit 6 g du "sel" préparé précédemment et 33 g d'isobutane contenant 2 g de n-butane (étalon interne). On pressurise avec de l'éthylène jusqu'à 0,9 MPa, pression que l'on maintient constante par ajout d'éthylène. Après 3 hr d'agitation 56% de l'isobutane initialement présent a été converti. Le produit de la réaction a été soutiré; il avait la composition suivante:
méthylbutane: 4,4 %
diméthyl-2,3 butane: 62,1 %
autres isohexanes: 5,9 %
triméthyl-2,2,4 pentane: 7 %
autres isooctanes: 12,3 %
alcanes supérieurs: 8,2 %

Le processus a été répété plusieurs fois.

### EXEMPLE N° 3

*Préparation du chlorhydrate de N-méthylimidazole* Dans un ballon à fond rond contenant 60 mL d'éther sec et 0,1 mole de N-méthymimidazole on fait buller lentement un courant d'acide chlorhydrique gazeux. Il se forme quantitativement un liquide jaune pâle, non soluble dans l'éther, qui cristallise après trois lavages avec de l'éther sec .

*Préparation d'un sel acide.* Dans une suspension de 11,6g de chlorure d'aluminium dans de l'heptane on ajoute 0,046 mole de chlorhydrate de N-méthylimidazole. On constate la formation en moins d'une heure d'une phase liquide non miscible dans l'heptane.

*Alkylation de l'isobutane par l'éthylène.* Dans un tube en verre à pression, maintenu à 20° C, on introduit 6 g du "sel" préparé précédemment et 33 g d'isobutane contenant 2 g de n-butane (étalon interne). On pressurise avec de l'éthylène jusqu'à 0,9 MPa, pression que l'on maintient constante par ajout d'éthylène. Après 3 hr d'agitation 26% de l'isobutane initialement présent a été converti. Le produit de la réaction a été soutiré; il avait la composition suivante:
méthylbutane: 4,5%
diméthyl-2,3 butane: 73 %
autres isohexanes: 7,8 %
triméthyl-2,2,4 pentane: 4,7 %
autres isooctanes: 7,8 %
alcanes supérieurs: 1,6 %

Le processus a été répété plusieurs fois.

### EXEMPLE COMPARATIF

Dans les conditions d'alkylation de l'exemple 1 on a utilisé le chlorure de N-butylpyridinium à la place du chlorhydrate de pyridine. La conversion n'était que de 39% pour une composition des produits voisine.

## Revendications

1. Procédé d'alkylation d'au moins une isoparaffine par au moins une oléfine en présence d'un catalyseur renfermant au moins un halogénure d'aluminium, caractérisé en ce que le catalyseur est constitué par un mélange liquide à la température de réaction d'au moins un halogénure d'aluminium avec au moins un halohydrate d'une amine, la fraction molaire d'halogénure d'ammonium étant supérieure à 0,5.

2. Procédé selon la revendication 1 dans lequel l'halogénure d'aluminium est choisi dans le groupe constitué par le chlorure d'aluminium et le bromure d'aluminium.

3. Procédé selon l'une des revendications précédentes dans lequel l 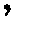 halohydrate d 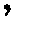 amine comporte une mole d 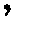 acide halohydrique par mole d 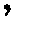 amine.

4. Procédé selon l'une des revendications précédentes dans lequel l 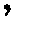 halohydrate d 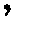 amine comporte deux moles d 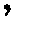 acide halohydrique par mole d 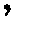 amine.

5. Procédé selon les revendications précédentes dans lequel les halohydrates sont choisis dans le groupe des chlorhydrates et des bromhydrates.

6. Procédé selon les revendications précédentes dans lequel l'amine est choisie dans le groupe constitué par les amines de formules générales: 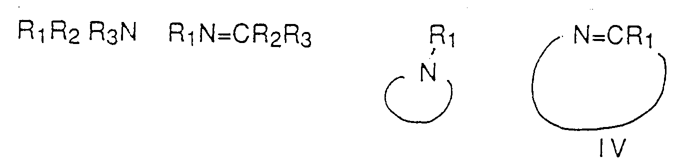 dans lesquelles R₁, R₂, R_{3,} identiques ou différents, représentent des restes hydrocarbyles et le composé IV appartient aux groupes des pyridines, des imidazoles, des triazines, des pyrazoles, des pyrimidines et des triazoles.

7. Procédé selon les revendications précédentes dans lequel R₁, R₂, R_{3,} identiques ou différents, sont constitués de radicaux alkyles, cycloalkyl, aryl, aralkyl ou alkaryl.

8. Procédé selon l'une des revendications précédentes dans lequel l'amine est choisie dans le groupe constitué par la pyridine, la picoline-4, le N-méthylimidazole.

9. Procédé selon les revendications précédentes dans lequel l'isobutane est alkylé par une oléfine choisie dans le groupe formé par l'éthylène, le propylène, les n-butènes, ou leurs mélanges.

10. Procédé selon les revendications précédentes dans lequel le catalyseur est réactivé par addition d 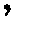 acide halohydrique et/ou d 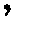 un halogénure d 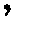 alkyle et/ou d 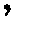 halogénure d 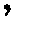 aluminium.

## Claims

1. A process for the alkylation of at least one isoparaffin by at least one olefin in the presence of a catalyst comprising at least one aluminium halide, characterized in that the catalyst is constituted by a mixture, which is liquid at the reaction temperature, of at least one aluminium halide with at least one amine hydrohalide, the mole fraction of the ammonium halide being more than 0.5.

2. A process according to claim 1, in which the aluminium halide is selected from the group constituted by aluminium chloride and aluminium bromide.

3. A process according to any one of the preceding claims, in which the amine hydrohalide comprises one mole of hydrohalic acid per mole of amine.

4. A process according to any one of the preceding claims, in which the amine hydrohalide comprises two moles of hydrohalic acid per mole of amine.

5. A process according to the preceding claims, in which the hydrohalides are selected from the group formed by formed by hydrochlorides and hydrobromides.

6. A process according to the preceding claims, in which the amine is selected from the group formed by constituted by amines with general formulae: 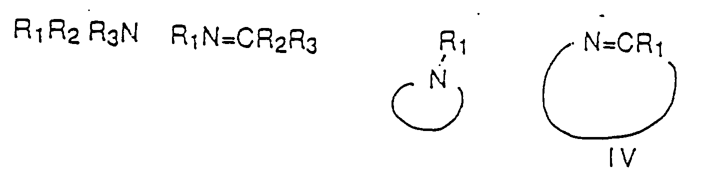 where R₁, R₂ and R₃, which may be identical or different, represent hydrocarbyl residues, and compound IV is a pyridine, imidazole, triazine, pyrazole, pyrimidine or triazole.

7. A process according to the preceding claims, in which R₁, R₂ and R₃, which may be identical or different, are constituted by alkyl, cycloalkyl, aryl, aralkyl or alkaryl radicals.

8. A process according to any one of the preceding claims, in which the amine is selected from the group formed by pyridine, 4-picoline and N-methylimidazole.

9. A process according to the preceding claims, in which the isobutane is alkylated by an olefin selected from the group formed by ethylene, propylene, n-butenes, and mixtures thereof.

10. A process according to the preceding claims,, in which the catalyst is reactivated by addition of hydrohalic acid and/or an alkyl halide and/or an aluminium halide.

## Patentansprüche

1. Verfahren zur Alkylierung von wenigstens einem Isoparaffin durch wenigstens ein Olefin in Anwesenheit eines Katalysators, der wenigstens ein Aluminiumhalogenid enthält, dadurch gekennzeichnet, daß der Katalysator durch eine flüssige Mischung bei der Reaktionstemperatur von wenigstens einem Aluminiumhalogenid mit wenigstens einem Halohydrat eines Amins gebildet wird, wobei die molare Fraktion von Ammoniumhalogenid größer als 0,5 ist.

2. Verfahren nach Anspruch 1, bei welchem das Aluminiumhalogenid aus der Gruppe ausgewählt wird, die durch das Aluminiumchlorid und das Aluminiumbromid gebildet ist.

3. Verfahren nach einem der vorhergehenden Ansprüche, bei welchem das Halohydrat von Amin ein Mol Halogenwasserstoffsäure pro Mol Amin umfaßt.

4. Verfahren nach einem der vorhergehenden Ansprüche, bei welchem das Halohydrat von Amin zwei Mol Halogenwasserstoffsäure pro Mol Amin umfaßt.

5. Verfahren nach einem der vorhergehenden Ansprüche, bei welchem die Halohydrate aus der Gruppe der Chlorhydrate und der Bromhydrate ausgewählt werden.

6. Verfahren nach den vorhergehenden Ansprüchen, bei welchem das Amin aus der Gruppe ausgewählt wird, die durch die Amine der allgemeinen Formeln 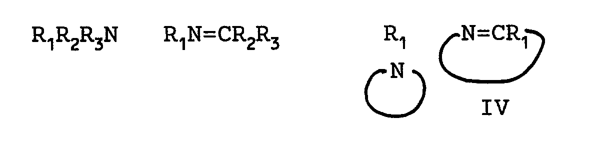 gebildet werden, in welchen R₁, R₂, R₃, identisch oder unterschiedlich, Hydrocarbylreste darstellen, und die Verbindung IV zu den Gruppen der Pyridine, der Imidazole, der Triazine, der Pyrazole, der Pyrimidine und der Triazole gehören.

7. Verfahren nach den vorhergehenden Ansprüchen, bei welchem R₁, R₂, R₃, identisch oder unterschiedlich, aus Alkyl-, Cycloalkyl-, Aryl-, Aralkyl- oder Alkarylresten gebildet werden.

8. Verfahren nach einem der vorhergehenden Ansprüche, bei welchem das Amin aus der Gruppe ausgewählt wird, die durch das Pyridin, das 4-Picolin, das N-Methylimidazol gebildet ist.

9. Verfahren nach den vorhergehenden Ansprüchen, bei welchem das Isobutan durch ein Olefin alkyliert wird, das aus der Gruppe ausgewählt ist, welche durch das Ethylen, das Propylen, die n-Butene oder deren Mischungen gebildet ist.

10. Verfahren nach den vorhergehenden Ansprüchen, bei welchem der Katalysator durch Addition von Halogenwasserstoffsäure und/oder von einem Alkylhalogenid und/oder von Aluminiumhalogenid reaktiviert wird.
